# EUROPEAN PATENT SPECIFICATION

(11) **EP 2 136 211 B1**
(45) Date of publication and mention of the grant of the patent: **07.08.2013**
(21) Application number: 08710396.6
(22) Date of filing: 15.02.2008
(51) Int. Cl.: G01N 33/543, G01N 33/68

(54) **DETERMINATION METHOD FOR ALLERGIC DISEASE**
BESTIMMUNGSVERFAHREN FÜR EINE ALLERGISCHE ERKRANKUNG
PROCÉDÉ DE DÉTERMINATION POUR UNE MALADIE ALLERGIQUE

(30) Priority: 12.03.2007 JP 2007061702
(43) Date of publication of application: 23.12.2009
(73) Proprietor: The University of Tokushima, Tokushima-shi Tokushima 770-8501 (JP)
(72) Inventor: KIDO, Hiroshi, Tokushima-shi Tokushima 770-8503 (JP); SUZUKI, Koichi, Tokushima-shi Tokushima 770-8503 (JP)
(74) Representative: Richards, William John
(86) International application number: PCT/JP2008/000242
(87) International publication number: WO 2008/111281

(56) References cited:
- WO-A1-2004/102194
- JP-A- 2000 009 728
- JP-A- 2004 028 953
- JP-A- 2005 017 153
- JP-A- 2006 267 063
- JP-A- 2006 519 025
- DATABASE WPI Week 200677 Thomson Scientific, London, GB; AN 2006-751207 XP002586799 & JP 2006 267063 A 5 October 2006 (2006-10-05)
- FALL B I ET AL: "Microarrays for the screening of allergen-specific IgE in human serum" ANALYTICAL CHEMISTRY 20030201 AMERICAN CHEMICAL SOCIETY US, vol. 75, no. 3, 1 February 2003 (2003-02-01), pages 556-562, XP002586802 DOI: DOI:10.1021/AC026016K
- SCHWEITZER B ET AL: "Inaugural article: immunoassays with rolling circle DNA amplification: a versatile platform for ultrasensitive antigen detection" PROCEEDINGS OF THE NATIONAL ACADEMY OF SCIENCES OF THE UNITED STATES (PNAS), NATIONAL ACADEMY OF SCIENCE, US LNKD- DOI:10.1073/PNAS.170237197, vol. 97, no. 18, 29 August 2000 (2000-08-29), pages 10113-10119, XP002207138 ISSN: 0027-8424
- KWIATKOWSKI M ET AL: "A high-capacity manifold support for the detection of specific IgE antibodies in allergic individuals" JOURNAL OF IMMUNOLOGICAL METHODS, ELSEVIER SCIENCE PUBLISHERS B.V.,AMSTERDAM, NL LNKD- DOI:10.1016/0022-1759(94)90218-6, vol. 168, no. 1, 12 January 1994 (1994-01-12), pages 137-143, XP023987109 ISSN: 0022-1759 [retrieved on 1994-01-12]
- FUSEK M ET AL: "IMMOBILIZATION AND NONSPECIFIC ADSORPTION OF PROTEINS TO PYROGENOUS HIGHLY DISPERSED SILICON DIOXIDE" BIOTECHNOLOGY LETTERS, vol. 9, no. 8, 1987, pages 561-566, XP002586800 ISSN: 0141-5492
- MARCH J G ET AL: "Fluorimetric determination of phytic acid based on the activation of the oxidation of 2,2'-dipyridyl ketone hydrazone catalysed by Cu(II)." THE ANALYST JUN 1999 LNKD- PUBMED:10736872, vol. 124, no. 6, June 1999 (1999-06), pages 897-900, XP002586801 ISSN: 0003-2654
- TAKESHI MATSUYA ET AL: "A new time-resolved fluorometric microarray detection system using core-shell-type fluorescent nanosphere and its application to allergen microarray" ANALYTICAL AND BIOANALYTICAL CHEMISTRY, SPRINGER, BERLIN, DE LNKD- DOI:10.1007/S00216-006-0455-9, vol. 385, no. 5, 20 May 2006 (2006-05-20), pages 797-806, XP019420274 ISSN: 1618-2650
- ROSEN STEFAN ET AL: "A cytoplasmic lectin produced by the fungus Arthrobotrys oligospora functions as a storage protein during saprophytic and parasitic growth" MICROBIOLOGY (READING), vol. 143, no. 8, 1997, pages 2593-2604, XP002589176 ISSN: 1350-0872
- LAZDINS J K ET AL: "In vitro effect of transforming growth factor-beta on progression of HIV-1 infection in primary mononuclear phagocytes" JOURNAL OF IMMUNOLOGY, AMERICAN ASSOCIATION OF IMMUNOLOGISTS, US, vol. 147, no. 4, 15 August 1991 (1991-08-15), pages 1201-1207, XP002509862 ISSN: 0022-1767
- RHEE SANG HOON ET AL: "Pathophysiological role of Toll-like receptor 5 engagement by bacterial flagellin in colonic inflammation" PROCEEDINGS OF THE NATIONAL ACADEMY OF SCIENCES OF THE UNITED STATES OF AMERICA, vol. 102, no. 38, September 2005 (2005-09), pages 13610-13615, XP002589177 ISSN: 0027-8424

## Description

### Technical Field

The present invention relates to a method for determining allergic diseases (food allergy, pollenosis, drug allergy, atopy, allergic asthma, allergic rhinitis, dermatitis, etc.) without collecting blood, andusingsaliva, nasaldischarge, tears, etc., which allergic diseases are said that be a folk disease where one out of three is said to have some allergic symptoms.

### Background Art

Various allergies are known, comprising food allergy caused by foods, dust allergy caused by trash and dust, allergy caused by house dust mite, pollenosis, contact dermatitis, metal allergy, drug allergy, and atopy. Among these, particularly food allergy is a harmful immune response caused by ingestion of allergy-causing substances (hereinafter referred to as food allergen) contained in foods, which induces dermatitis, asthma, digestive disorder, anaphylactic shock, etc. As the number of such food allergy patients is increasing, serious problems are arisen in the field of medicine and food industry. These harms may lead to death, and preventive measures should be taken.

Further, recently, blood, urine, etc. are used as general clinical test sample, while the pain and physical stress caused by blood collection is becoming a serious problem particularly in newborns, infants, critically ill patients, and patients to whom blood collection is difficult. Therefore, as one challenge in the next generation medical treatment, the necessity of noninvasive clinical test is pointed, and challenges will soon start across the world. As blood collection involves physical pain and stress to a patient, infection risk of AIDS, hepatitis type B and C, and risk of accidental injection by medical personnel, development of a safe test method using body fluid instead of blood is awaited. Specifically, urine, saliva, nasal discharge, tears, vaginal fluid, intestinal fluid, etc. may substitute blood, while the immediate obj ect is said to be the technical development with the use of urine, saliva, nasal discharge, and tears which are generally easy to collect with few pain.

Allergy is a condition that affects the body when a biophylaxis substance (IgE, IgG, IgA, IgM, etc.) that prevents an antigen recognized as a foreign substance to enter in the body acts excessively beyond biological defense. These biophylaxis substances (IgE, IgG, IgA, IgM, etc.) are secreted in mucosal membrane which is a site of entrance for a foreign substance, besides blood.

Among urine, saliva, nasal discharge, and tears which are easy to collect with less pain, particularly saliva which reflects mucosal environment is drawing attention. Saliva is a body fluid secreted from large salivary gland (parotid gland, submandibular gland, sublingual gland) and minor salivary gland, being transparent and viscous, and its physical properties are: specific gravity of 1.000 to 1.008 g/mL, pH 6.2 to 7. 6; and the composition includes 99.5% water; organic components (amylase, maltase, mucin, serum albumin, serum globulin, uric acid, urea, etc.); and inorganic components (Na, K, Cl, sodium bicarbonate, calcium carbonate, calcium phosphate, sodium phosphate, etc.). It has been attempted from about 20 years ago to analyze saliva for use in diagnosing or screening diseases, while it is still not common as for blood or urine.

The advantages of saliva include that it can be collected with less pain, is noninvasive, and is not influenced by the age, or physical or mental handicap of a patient, and that can be easily collected repeatedly. As saliva reflects well the blood condition, it is thought to be suitable for monitoring the general condition. Further, saliva contains, as an allergy-related biophylaxis substance secreted by mucosal membrane, dimeric secretory IgA (sIgA), IgG, and a minute amount of IgE. As its antibody activity and antibody level reflect the allergic conditions of the mucosal membrane, it is necessary to determine the allergic conditions in a comprehensive manner by measuring these levels.

In a noninvasive method for determining allergy using saliva, etc. as a sample, it is an important information for diagnosing and treating allergy to estimate an antigen which is thought to be directly associated with allergy, by measuring the immune status of the mucosal membrane specific to allergy antigen, using a method that does not involve stress and pain to a patient. Therefore, a method for differentiating the severity of allergy by measuring secretory sIgA and blood IgE specific to allergy antigen of an allergic patient, and using the relative relation between these levels as an index (see for example, Patent Document 1); and a method for measuring the presence of food allergy or food intolerance in a patient, comprising measuring the antibody concentration against food allergen which is present in food, in a mucosal membrane sample from a patient such as saliva, and comparing the measured concentration with the normal concentration of the antibody in the mucosal membrane sample (see for example, Patent Document 2) are known.

On the other hand, the present inventors have previously reported a method for determining allergic diseases that enables determination of expansion and reduction patterns of allergens, and/or expansion and reduction patterns of allergen epitopes; a method for determining allergic diseases that enables detection of an allergen-recognizing antibody in a sample by an immunoassay using a Cy3 labeled secondary antibody, which method comprises reacting a diamond/DLC (Diamond-like Carbon) chip with an activating reagent, conducting a coupling reaction to a peptide comprising allergen protein or allergen epitope, subsequently contacting a sample with the allergen epitope determination chip to which blocking operation of unreacted active groups has been conducted, and detecting an allergen-recognizing antibody in the sample captured by the allergen epitope determination chip by immunoassay (see for example, see Patent Document 3).

Patent Document 1: Japanese Laid-Open Patent Application No. 2002-303628
Patent Document 2: US 6858398
Patent Document 3: Japanese Laid-Open Patent Application No. 2006-267063

### Disclosure of the Invention

### Object to be solved by the Invention

Many of the conventional techniques for determining allergic diseases were a determination method comprising measuring allergen-specific IgE antibody levels in blood, and sIgA in body fluid representing the allergic conditions. However, the measuring results of these tests did not reflect the clinical symptoms yet, and it is necessary to understand more specifically the clinical symptoms with a multilateral diagnosis of antibodies other than IgE (various IgGs, IgAs, sIgAs, etc.). The method for determining allergic diseases of the above-mentioned Patent Document 3, is an extremely superior method that enables to understand more specifically the clinical symptoms by conducting multilateral and global analysis (measurement of 20 to 60 or more antigens at one time) with a minute amount of sample such as 10 µL or less, including not only IgE but other antibodies (various IgGs, IgAs, sIgAs, etc.). However, the concentration of biophylaxis substances (IgE, IgG, IgA, IgM) in body fluid is often lower than that in blood. Therefore, when directly applying this method to the cases using noninvasive samples such as saliva instead of serum, it has been revealed that there is a problem that the analysis accuracy is inferior compared to when serum is used as a sample. The object of the invention is to provide a method for determining allergic diseases that enables a multilateral and global analysis with a minute amount of sample, even when body fluid other than blood such as saliva is used as a sample, which method particularly suppresses nonspecific reactions as much as possible, and enables measurement at a high sensitivity and with accuracy.

### Means to solve the object

The present inventors have made a keen study to solve the above object, and have reviewed all unit operations in order to realize a high sensitive measurement system which suppresses nonspecific reactions as much as possible. They found out that a high sensitivemeasurement systemwhich suppresses nonspecific reaction as much as possible can be realized, by blending glycine to the washing solution and by blending glycine and poly ethylene glycol to the blocking solution used for the washing and blocking operation in the steps for preparing allergen determination chip. Further, food residues may be contaminated into saliva, when meal or lactation is taken just before saliva collection. These may induce increase of background signal, and decrease of measurement sensitivity in a measurement of allergen-specific antibody levels using protein chip. In order to solve such problems, and to maintain a constant and stable high sensitivity by using any sample, they used saliva etc. which has undergone pressure filtration using a low protein-adsorbing filter, and realized a high sensitive measurement system which suppresses nonspecific reactions as much as possible, and confirmed that a noninvasive determination of allergic diseases is possible. The present invention has been thus completed.

Specifically, the present invention relates to:
(1) a method for determining allergic diseases according to claim 1;
(2) the method for determining allergic diseases according to (1), wherein a potassium chloride-containing solution is used as a sample diluent;
(3) the method for determining allergic diseases according to (1) or (2), wherein a potassium chloride-containing solution is used as a diluent of a labeled-secondary antibody;
(4) the method for determining allergic diseases according to any one of (1) to (3), wherein a Cy3 labeled-secondary antibody is used as a labeled-secondary antibody;
(5) the method for determining allergic diseases according to any one of (1) to (4), wherein saliva, tears or nasal discharge is used as a sample;
(6) the method for determining allergic diseases according to wherein saliva, tears or nasal discharge which turbidity at an absorption wavelength of 600 nm is 25 or less is used as a sample;
(7) the method for determining allergic diseases according to (5), wherein saliva, tears or nasal discharge which has undergone pressure filtration with a low protein-adsorbing filter is used as a sample;
(8) the method for determining allergic diseases according to any one of (1) to (7), wherein the allergen is a food allergen.

Further, the present invention relates to: (10) an allergy determination chip according to claim 9

Moreover, the present invention relates to:
(10) a kit for determining allergic diseases comprising the allergy determination chip according to (9), and a labeled secondary antibody
(11) the kit for determining allergic diseases according to (10) wherein the labeled secondary antibody is one or more labeled secondary antibody selected from the group consisting of a labeled anti-sIgA antibody, labeled anti-IgG antibody, labeled anti-IgG1 antibody, labeled anti-IgG4 antibody, labeled anti-IgA antibody and labeled anti-IgE antibody;
(12) the kit for determining allergic diseases according to (10) or (11), further comprising a diluent containing potassium chloride for diluting a sample and/or for diluting a labeled secondary antibody;
(13) the kit for determining allergic diseases according to any one of (10) to (12), further comprising a low protein-adsorbing filter for pretreating a sample.

### Brief Description of the Drawings

[Fig. 1]
   It is a figure showing a protocol for measuring allergen-specific antibody.
[Fig. 2]
   It is a figure showing the comparison results of the turbidity and the background fluorescence intensity of saliva before and after filter treatment: (a) turbidity of saliva before and after filter treatment; and (b) background fluorescence intensity at the time of the allergen-specific antibody level measurement before and after filter treatment. The filter treatment decreased both turbidity and background fluorescence intensity. It has been shown that the decrease of measurement sensitivity can be suppressed by the filter treatment.
[Fig. 3]
   It is a figure showing results of the decrease of background fluorescence and the increase of fluorescence by the reaction with the allergen, with the addition of 0.1 M glycine to the washing solution and blocking solution: (a) comparison of background fluorescence intensity; and (b) comparison of fluorescence intensity measured by the reaction with the allergen; both before and after the addition of 0.1 M glycine. With the addition of glycine, the background fluorescence decreased, and the fluorescence measured by the reaction with the allergen increased by around 30%.
[Fig. 4]
   It is a figure showing the decreased results of background fluorescence with the addition of 10% PEG to the washing solution and blocking solution. The comparison of background fluorescence intensity, before and after the addition of 10% PEG is shown. The background fluorescence intensity decreased about 10% with the addition of PEG.
[Fig. 5]
   It is a figure showing the comparison results of background fluorescence intensity, before and after the addition of 0.3 M KCl. By comparing before and after the addition of 0.3 M KCl, when serum was reacted, the fluorescence intensity of the antibody reacting nonspecifically to the background decreased by 30 to 40%. The relative increase of the sensitivity was confirmed.
[Fig. 6]
   It is a figure showing the investigation results of optimization conditions of the allergen concentration to spot. OVM was spotted at a concentration of 5 to 100 fmol/spot, and saliva was diluted 1 to 100 fold for reaction. It has been revealed that the antibody content in saliva could be quantified when the antigen level was of a concentration of 50 fmol or more, within the dotted box. When the antigen level is a small amount such as 25 fmol or less, the antigen was not possible to capture all of the antibodies in the sample, and it was shown that acute measurement was not made.
[Fig. 7]
   The optimum dilution rate of saliva was measured from the change of dilution rate of saliva and fluorescence intensity, when the saliva sample was reacted at a dilution rate of 1 to 100 fold, under a condition of antigen concentration of 75 fmol, which is within the range of optimum antigen concentration. Under the condition of 5 to 50-fold diluted saliva, a high quantitative property was obtained, and it has been suggested to dilute saliva 5 to 50 fold for measurement.
[Fig. 8]
   It is a figure showing the investigation results with sIgA for saliva samples (sa001 - sa0027) from27foodallergy-patients. Various allergen components of milk and egg were spotted on a Gene Slide. Saliva samples from child patients having food allergy were diluted 10 fold, and subjected to reaction. Cy3 labeled secondary antibodies were used for detection.
[Fig. 9]
   It is a figure showing the detection results of food allergen-specific IgE antibodies in saliva. Various allergen components of milk and egg were spotted on a Gene Slide. Saliva samples from child patients having food allergy were diluted 5 fold, and subjected to reaction. Cy3 labeled secondary antibodies (Anti-Human IgE) were used for detection.
[Fig. 10]
   It is a figure showing the detection results of food allergen-specific IgG antibodies in saliva. Various allergen components of milk and egg were spotted on a Gene Slide. Saliva samples from child patients having food allergy were diluted 5 fold, and subjected to reaction. Cy3 labeled secondary antibodies (Anti-Human IgG) were used for detection.
[Fig. 11]
   It is a figure showing the measurement results of allergen-specific sIgA antibody level contained in nasal discharge and tears. Nasal discharge and tears were collected from patients showing allergic reactions to white birch pollen and apples. Nasal discharge from 2 healthy subjects was also collected. Nasal discharge was diluted 20 fold, and tears were diluted 10 fold to undergo a reaction on a protein chip to which antigen proteins of white birch pollen, apple, egg and milk were spotted. Cy3 labeled secondary antibodies (Anti-Human IgA) were used for detection.
[Fig. 12]
   One example of diagnosis result shown on a Radar chart with sIgA test levels of milk and egg allergy of a saliva sample, and IgE, IgA, IgG, IgG1, IgG4 test levels of blood is shown. Various allergen-specific antibody levels in blood and saliva (IgE, IgA, IgG, IgG1, IgG4, sIgA) were evaluated relatively and systematically with a Radar chart (diamond graph) using relative levels (0-200) for each concentration, to use as an indication to understand the disease conditions.

### Best Mode for carrying out the Invention

A method for determining allergic diseases of the present invention is not particularly limited as long as it is a method comprising activating a chemically modified diamond/DLC (Diamond-like Carbon) chip with an activating reagent; conducting a coupling reaction with a peptide containing allergen or allergen epitope; contacting a sample to the allergen determination chip to which washing and blocking operations of unreacted active groups have been conducted; and detecting an allergen recognizing antibody in the sample captured by the allergen determination chip by an immunoassay using a labeled secondary antibody; wherein a glycine-containing solution is used for the washing solution and a glycine and polythylene glycolcontaining solution is used as the blocking solution used in the above washing and blocking operations. The above sample is not particularly limited and examples include blood (serum), urine, saliva, nasal discharge, tears, vaginal fluid and intestinal fluid, while samples such as saliva, nasal discharge and tears, which antibody concentration is thought to be low are preferred from the view point that the effect of the present invention can be particularly conferred. Further, according to a method for determining allergic diseases of the present invention, when using a sample other than serum, such as saliva, it is possible to determine the expansion and reduction patterns of allergen and/or expansion and reduction patterns of allergen epitope.

The above allergen is not particularly limited as long as it has an immunogenicity, and even it is an haptene, if it generates immunogenicity by being bound with a peptide, it can be used as an allergen peptide of the present invention. Preferred examples of food allergen include eggs, milks, meats, fish, shellfishes and mollusks, cereals, beans and nuts, fruits, vegetables, beer yeast and gelatine. Among these, αs1-casein, αs2-casein, β-casein, κ-casein, α-lactoalubumin, β-lactoglobulin as main components of milk allergen; ovomucoid, ovoalubumin and conalubumin as main components of egg white allergen; gliadin as a main component of wheat allergen; proteins having a molecular weight of 24 kDa and 76 kDa which are the main proteins of buckwheat noodle; and Ara h1 which is the main protein of peanut can be preferably exemplified. Particularly, it is preferred to use one or more, preferably 8 types of allergens selected from the group consisting of sodium casein, α-casein, β-casein, κ-casein, α-lactoalubumin, β-lactoglobulin, ovomucoid, ovoalubumin, and conalubumin.

As a peptide comprising an allergen epitope of the present invention, chemically modified peptides such as sugar chain-modified peptide, phosphorylated peptide, acylated peptide, acetylated peptide, methylated peptide, and ubiquitinated peptide can be used. Such chemically modified peptide may be a natural chemically modified peptide, or an artificial chemically modified peptide. When using an epitope-containing peptide added with at least 2 amino acids at N' end side and/or C' end side of the peptide moiety having for example a size of 7 to 15 amino acids binding to MHC class II molecules as the above mentioned peptide comprising an allergen epitope, it is preferred from the point that it reacts with the patient' s antibody with a high sensitivity of several to several dozen folds. A peptide comprising an allergen epitope such as an epitope-containing peptide added with at least 2 amino acids at N' end side and/or C' end side of the peptide moiety binding to MHC class I I molecules maybe prepared by peptide synthesis, but it can be also prepared as a protease degrading peptide comprising an allergen epitope. Examples of such protease include trypsin, chymotrypsin, chathepsin and lysylendopeptidase. Particularly, when the allergen is a food allergen, trypsin degrading peptide can be preferably exemplified as a protease degrading peptide.

The above chemically modified diamond/DLC chip is not particularly limited as long as it is a diamond/DLC chip which is a substrate such as silicon, glass, stainless and plastic, coated with a diamond or diamond-like carbon (DLC), and that has been chemically modified so that an allergen peptide of an allergen or a peptide comprising an allergen epitope (hereinafter may be referred to as "allergen peptide") can be bound by a coupling reaction, etc. Examples of such chemically modified (diamond/DLC chip) include a diamond/DLC chip wherein the surface of the diamond/DLC chip has been for example chlorinated, aminated, carboxylated, etc. by chlorination, ammonia treatment, dicarboxylation, etc. Among these, a carboxylated diamond/DLC chip can be preferably exemplified.

The above mentioned chemically modified diamond/DLC chip is subjected to activation with an activating reagent. For example, as a method for immobilizing an allergen peptide of an allergen to a carboxylated diamond/DLC chip, it is preferred to use a carboxyl group (-COOH group) introduced to the base top, and to immobilize an allergen peptide having an amino group (-NH₂ group) by covalent binding using 1-Ethyl-3-(3-dimethylamino Propyl)-carbodiimide, hydrochloride (WSCD.HCl) or N-Hydroxy-succinimide (NHS) or other chemical cross-linkers. The outlines of chip activation and coupling reaction are shown in Fig. 13 of the above-mentioned Patent Document 3.

The above-mentioned chip after activation is washed with MilliQ water (extra pure water), and the coupling reaction with a peptide (fixing operation) is conducted. As a pretreatment of the immobilization operation of the peptide, sufficient humidity and drying are important factors for the immobilization level of the peptide and uniformity of the immobilization level. Therefore, it is preferred to conduct a drying treatment in vacuo for 1 hour with a vacuum desiccator. Further, a coupling reaction of peptides can be conducted by spotting an allergen peptide solution to an activated chip, preferably a chip that has been dried, and incubating the same for example at 37°C for 3 hours. It is preferred to use a solution wherein an allergen peptide has been diluted to a solution containing dimethyl sulfoxide (DMSO) or polyethylene glycol (PEG) as an allergen peptide solution, as the antibody binding level increases. In the present invention, it is necessary to use a glycine-containing solution for a washing solution and a glycine and polyethylene glycol-containing solution for a blocking solution used in the washing and blocking operations of unreacted active groups after the coupling reaction of an allergen peptide, in order to suppress as much as possible nonspecific reactions in the reaction of allergen epitope or allergen with the antibody in the sample (primary antibody). The glycine concentration of the glycine-containing solution is 0.01 to 0.5 M, preferably 0.05 to 0.5 M, and more preferably 0.1 to 0.2 M. For example, a buffer solution such as PBS (phosphate buffered saline) in which glycine is diluted is preferably used as a washing solution. The polyethylene glycol concentration of the glycine and polyethylene glycol-containing blocking solution is preferably 1 to 30%, more preferably 5 to 20%, and most preferably 8 to 12%. Preferred examples of blocking solution include BSA solution in which glycine and polyethylene glycol are diluted. By using such washing solution and blocking solution in combination to perform washing and blocking operations of unreacted active groups, the background can be decreased, and the fluorescence intensity and coloring intensity can be increased, to improve the measurement sensitivity by about 35%.

Further, it is preferred to use a washing solution further containing polyethylene glycol beside glycine in the above washing operation. The polyethylene glycol concentration of the glycine and polyethylene glycol-containing washing solution solution is preferably 1 to 30%, more preferably 5 to 20%, and most preferably 8 to 12%. For example, a buffer solution such as PBS in which glycine and polyethylene glycol are diluted is preferably used as a washing solution. By using such washing solution and blocking solution, preferably in combination, to perform washing and blocking operations of unreacted active groups, the background can be decreased, and the fluorescence intensity and coloring intensity can be relatively increased, to improve the measurement sensitivity.

By removing by aspiration the blocking solution according to need after the washing and blocking operations with an aspirator, the allergen determination chip of the present invention can be prepared. Specifically, the allergen determination chip of the present invention is characterized by that a chemically modified diamond/DLC (Diamond-like Carbon) chip is activated with an activating reagent, a coupling reaction with a peptide comprising an allergen or allergen epitope is conducted, and then the washing and blocking operations of unreacted active groups have been performed using a washing solution comprising glycine and blocking solution comprising glycine and PEG, preferably a washing solution and blocking solution comprising glycine and polyethylene glycol. A sample is then applied to the allergen determination chip.

As it is stated in the above, a sample in the method for determining allergic diseases of the present invention is preferably a nonhematological component such as saliva, tears, nasal discharge, and urine, and it is preferred that saliva, etc. undergo a pretreatment. The pretreatment is preferably conducted so that a turbidity of a body fluid such as saliva at a absorption wavelength of 600 nm is 25 or less, preferably 20 or less, and more preferably 15 or less in order to enhance the measurement sensitivity. Specifically, it may be a centrifugal treatment, while a method of conducting a pressure filtration with a low protein-adsorbing filter is particularly preferred. Also disclosed herein is a noninvasive method for determining allergic diseases comprising using saliva, tears or nasal discharge which has undergone a pressure filtration with a low protein-adsorbing filter as a sample. By a pressure filtration with a low protein-adsorbing filter, the turbidity of a body fluid such as saliva, tears and nasal discharge at an absorption wavelength of 600 nm is made to 25 or less, and by applying the sample on the allergen determination chip of the present invention, various allergens can be analyzed at a high sensitivity by suppressing nonspecific adsorption. As a low protein-adsorbing filter, a commercialized product can be used, and examples include MILLEX GV Filter Unit 0.22 µm filter (MILLIPORE), Filter insert (size 0.2) (TreffLab) and SEITZ depth filter (Pall Corporation). Further, by using a potassium chloride (KCl)-containing solution as a diluent of these samples, the background can be decreased and the fluorescence intensity and coloring intensity can be relatively increased, to improve the measurement sensitivity. The potassium chloride concentration is preferably 0.1 to 0.5 M, preferably 0.2 to 0.4 M.

An immunoassay in the determination method of the present invention is not particularly limited as long it is a known immunoassay that can detect an allergen recognizing antibody in the sample captured by a determination chip, while ELISA using a labeled-secondary antibody is preferred. Examples of labeled secondary antibody include fluorescence-labeled secondary antibodies such as Cy3, Cy5, FITC and Rhodamin; enzyme-labeled secondary antibodies such as peroxidase and alkaline phosphatase; magnetic beads-labeled secondary antibodies and infared labeled secondary antibodies. Among these, Cy3 labeled-secondary antibody can be preferably exemplified. Further, as a secondary antibody, one or more labeled secondary antibody selected from the group consisting of labeled anti-sIgA antibody, labeled anti-IgG antibody, labeled anti-IgG1 antibody, labeled anti-IgG4 antibody, labeled anti-IgA antibody, and labeled anti-IgE antibody can be preferably exemplified. By using a potassium chloride (KCl)-containing solution for a diluent of these labeled secondary antibodies, the background can be decreased, and the fluorescence intensity and coloring intensity can be relatively increased, to improve the measurement sensitivity. The potassium chloride concentration is 0.1 to 0.5 M, and preferably 0.2 to 0.4 M.

A kit for determining allergic diseases of the present invention is not particularly limited as long as it is a kit comprising the above allergen determination chip of the present invention, and a labeled secondary antibody. Examples of labeled secondary antibody include the above labeled secondary antibodies, specifically including one or more labeled secondary antibody selected from the group consisting of labeled anti-sIgA antibody, labeled anti-IgG antibody, labeled anti-IgG1 antibody, labeled anti-IgG4 antibody, labeled anti-IgA antibody, and labeled anti-IgE antibody, labeled with fluorescence or enzyme. Further, it is preferred that the kit for determining allergic diseases comprises a diluent comprising potassium chloride for diluting the sample and/or diluting the labeled secondary antibody, or a low protein-adsorbing filter for pretreating the sample.

As a secondary antibody in the method for determining allergic diseases or in the kit for determining allergic diseases of the present invention, Fab fragment or F(ab')₂ fragment, etc. of the antibody can be used. For example, Fab fragment can be prepared by treating the antibody with papain, and F(ab')₂ fragment can be prepared by treating the antibody with pepsin, etc.

In the method for determining allergic diseases disclosed herein, by using 1 to 5 µL of sample solution, preferably saliva which has undergone pressure filtration with a low protein-adsorbing filter, an antibody in the sample can be quantified, and 10 or more types of allergens, preferably 25 or more, for example 25 to 60 types of allergens can be quantified at once under the same conditions. Thus, the difference of reactivity of sIgA and IgG in the same patient, or expansion and reduction patterns of the allergen, and/or expansion and reduction patterns of the allergen epitope can be determined.

In the following, the present invention will be explained in details by referring to the Examples, while the technical scope of the present invention will not be limited to these exemplifications.

### Example 1

### [Experiment materials]

Gene Slide DLC (Toyo Kohan CO., Ltd.), 384-well flat bottom-microplate (CORNING), reaction plate (SANPLATEC) and MILLEX GV Filter Unit 0.22 µm (MILLIPORE) were purchased for use. Ovoalubumin (OVA) (SIGMA), ovomucoid (OVM), conalubumin (SIGMA), α-casein (SIGMA), β-casein (SIGMA), α-lactoalubumin (SIGMA), β-lactoglobulin (SIGMA), Human Serum Immunoglobulins G, A and M (67/086) (NIBSC), Goat anti-human IgA (ZYMED), and Cy3 ™ Protein ArreyDyePack (Amersham Biosciences) were purchased for use. Further, 0.1 M potassium phosphate buffer solution (KPB) pH 6.0/30% PEG, 0.1 M glycine in PBS, 10 mg/mL Bovine serum albumin (BSA)/0.1 M glycine 10% PEG, 10 mg/mL BSA/0.05% Tween 20/0.3 M KCl, 50 mM Tris-HCl (pH 7.5), 150 mM NaCl and 0.05% Tween20 (TTBS) were used.

### [Measurement procedures]

Gene Slides (R) which have been activated have been purchased, and antigen proteins were diluted to a concentration of 10 to 75 fmol/mL with 0.1 M potassium phosphate buffer solution (KPB) (pH 6.0) /30% PEG. Each solution was applied on a 384-well plate, and spotted in an amount of 1 nL on a slide with GeneMachines (OmmniGrid Accent; NIPPN TechnoCluster, Inc). Further, the slides were dried at 37°C under light shielding for 1 hour, to immobilize the antigen. Similarly, a standard solution (Human Serum Immunoglobulins G, A and M (67/086)) was diluted serially (0.24 to 3.12 unit/mL), spotted and immobilized on a slide.

### [Blocking reaction of unreacted active groups]

The slides were transferred to a reaction plate, added with 8 mL of washing solution (0.1 M glycine in PBS), shaken for 5 minutes, and the washing solution was removed by aspiration with an aspirator (VARIABLE SPEED PUMP; BIORAD). After washing 3 times in a similar manner, it was further washed twice with purified water. Centrifugation was conducted with a centrifuge (1000 rpm x 1 min) to remove water droplets on the slide surface. Blocking reagent (10 mg/mL BSA/0.1 M glycine) was added by 20 µL/block, allowed to rest in cold storage (4°C) under light shielding for an overnight reaction.

### [Capture reaction of allergen-specific antibody]

Blocking reagent was removed by aspiration with an aspirator, and the sample was diluted appropriately with an antibody diluent (10 mg/mL BSA/0.05% Tween 20/0.3 M KCl) (about 5 to 20 fold dilution). Samples diluted in each block (primary antibody) were sampled 20 µL each, and allowed to rest at 37°C under light shielding for 1 hour.

### [Reaction with a secondary antibody]

The primary antibody solution was removed by aspiration with an aspirator, and the slides were transferred to a reaction plate. 8 mL of washing solution (50 mM TTBS) was added, shaken for 5 minutes, and the washing solution was removed by aspiration. After washing 3 times in a similar manner, it was further washed twice with purified water. Water droplets on the slide surface were removed by Spin down (1000 rpm, 1 min) with a centrifuge, 20 µL/block of secondary antibody (prepared to 1.5 to 6 µg/mL) diluted with 10 mg/mL BSA/0.05% Tween20/0.3 M KCl was added, and allowed to rest at 37°C under light shielding.

### [Detection of antibody captured by an antigen]

The secondary antibody solution was removed by aspiration, the slides were transferred to a reaction plate, 8 mL of 50 mM TTBS was added, shaken for 5 minutes and the washing solution was removed by aspiration. After washing 3 times in a similar manner, it was further washed twice with purified water. Water droplets on the slide surface were removed by Spin down (1000 rpm 1 min) with a centrifuge. Fluorescence intensity was measured (Ex: 532 nm, Em: 570 nm) with a fluorescence scanner (FLA-8000; FUJIFILM) and the measurement level was calculated from the calibration curve of the standard solution which was measured at the same time.

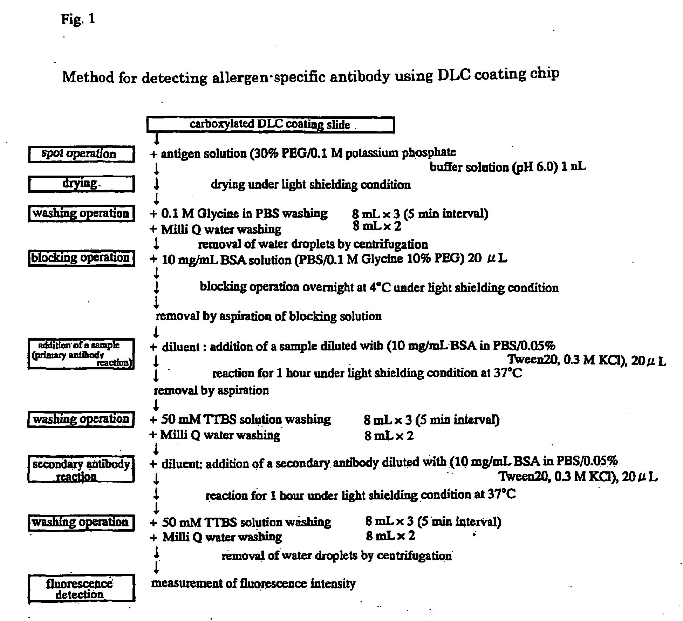

The protocol is shown in Fig. 1.

### Example 2

### [Sample collection]

Saliva collection was performed by letting a subject bite a sponge and transferring saliva into a collection recipient. Specifically, a cylindrical sponge was introduced into the mouth, and bit for about 45 seconds, paying attention to accidental ingestion, and dribble was absorbed. Then saliva was squeezed from the sponge removed from the mouth and collected in the recipient. The collected sample was stored in a frozen state (-20°C or under) until measurement.

### [Pretreatment of saliva sample]

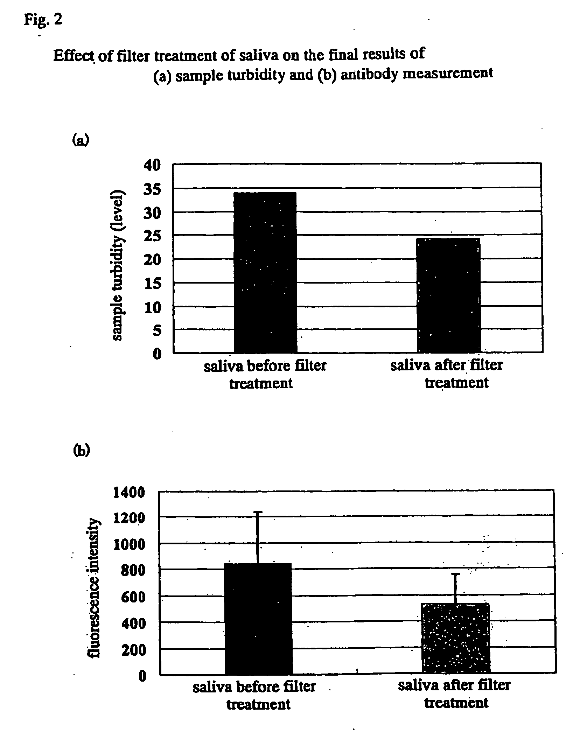

Saliva was subjected to pressure filtration with a low protein-adsorbing filter of 0. 45 to 0. 2 micron (MILLEX GV Filter Unit 0.22 µm (MILLIPORE) filter) to eliminate influence from food residues. For the elimination level of food residues, the measurable standard level was assessed by turbidity (see Fig. 2(a)), and for each sample, the background fluorescence intensity was measured under the conditions at the time of allergen-specific antibody level measurement (see Fig. 2(b)). As a result, turbidity of saliva decreased from 33.8 before filter treatment to 24.2 after filter treatment, and the background fluorescence intensity at the time allergen-specific antibody levels measurement was decreased, and it was shown that the filter treatment suppresses the decrease of measurement sensitivity. Turbidity was measured with F-2000 (HITACHI) devices, and the absorption wavelength was measured at 600 nm.

### Example 3

### [Conditions for suppressing nonspecific reaction necessary to measure allergen-specific antibodies in saliva]

### 1. Use of glycine and polyethylene glycol in the washing solution and blocking solution for suppressing nonspecific reactions

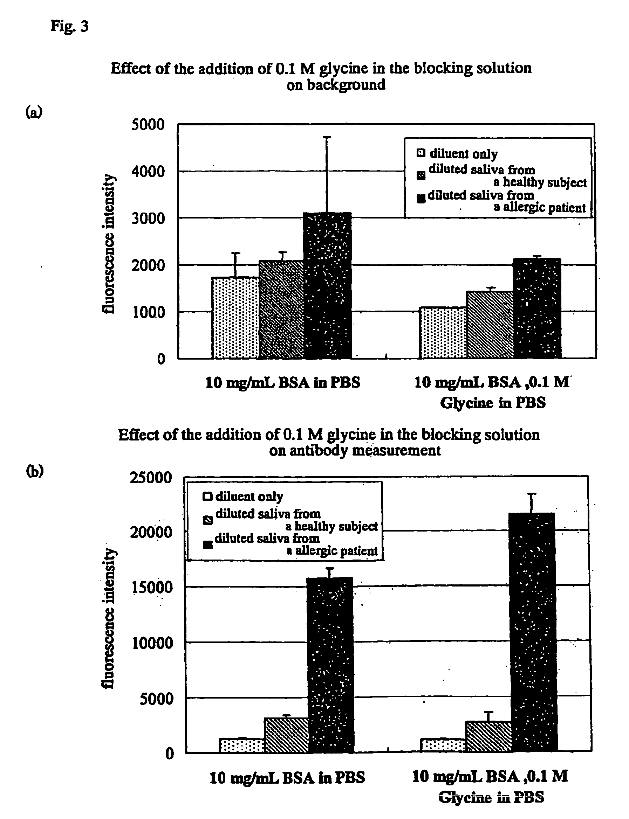

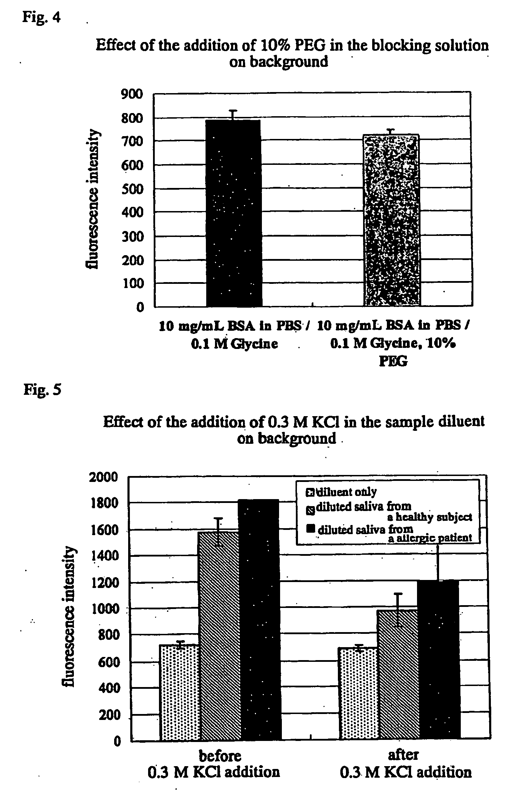

Conventionally, a solution comprising Tris (Tris-HCl) was used for washing or blocking after immobilizing antigen protein. A solution added with 0.1 M glycine was used for the washing solution and blocking reagent after immobilizing the antigen protein in order to suppress nonspecific reactions. Glycine is an amino acid with the smallest molecular weight, and which influence on the steric structure of the immobilized antigen protein is estimated to be small. As a result, the background signal (fluorescence intensity) was decreased about 30 to 40%, and the relative fluorescence intensity measured in the antigen antibody reaction with the allergen increased. Further, the reactivity of the antibody bound to the antigen was also increased. From the above decrease of the background, and the increase of the antibody reactivity, the fluorescence intensity of the antibody reacting with the antigen increased by about 30%, which increased the measurement sensitivity (see Fig. 3). Further, with the hint that the fluorescence intensity around the antigen protein added with polyethylene glycol (PEG) was significantly kept low at the time of spotting, PEG was added to the blocking reagent. As a result, the background fluorescence was further suppressed and the high sensitivity was achieved (see Fig. 4).

### 2. Example of nonspecific reaction suppression by the addition of 0.3 M KCl salt to saliva or serum, and secondary antibody reaction solution

Diluted saliva or serum of a normal subject, saliva or serum of an allergic patient, and only diluent (buffer) were reacted for 1 hour on a carboxylated DLC chip, then 6.0 µg/mL of Cy3-labeled anti-human IgE was added and reacted for 30 minutes . For the diluent used, a solution in which 0.3M KCl was added and not added to 10 mg/mL BSA in PBS/0.05% Tween20 was prepared, and each of the background fluorescence intensity was compared. By adding 0.3 M KCl to the diluent for saliva or serum, and secondary antibody reaction, the fluorescence intensity by nonspecific reaction was suppressed 30% to 40% lower (Fig. 5 for reference shows an example of serum which nonspecific reaction is relatively strong).

### Example 4

### [Test of optimization conditions of antibody concentration and antigen concentration for measuring specific antibody in saliva]

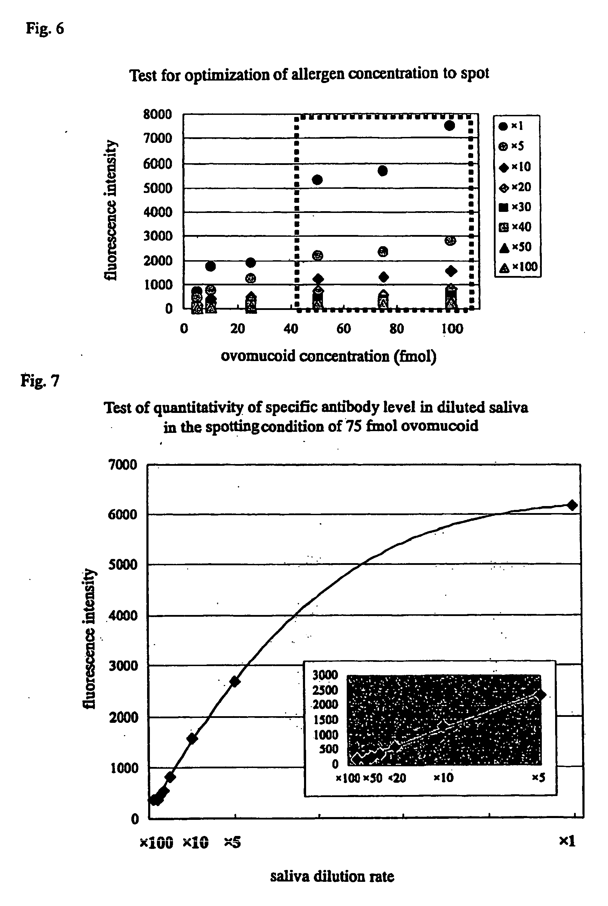

In order to obtain the optimum concentration for spotting, OVM was spotted within the concentration of 5 to 100 fmol/spot, and the reaction was carried out on the saliva sample at a dilution rate of 1 to 100 fold. Under a condition in which saliva is diluted 1 to 100 fold, it has been revealed that the antibody content in saliva can be quantified, when the antigen level on DLC chip is of a concentration of 50 fmol or more, within the dotted box. When the antigen level on DLC chip is 25 fmol or less, the antigen cannot capture all of the antibodies in saliva, and it has been revealed that the antibody content in the sample cannot be measured accurately (see Table 1 and Fig. 6). Under a condition in which 75 fmol antigen within the optimum antigen concentration range is applied on a DLC chip, saliva was tested by being diluted 1 to 100 fold (see Fig. 7), and a high quantitativity was obtained in the measurement of antibody level under a dilution condition of 5 to 50 fold, and the dilution rate of saliva at the time of measurement was determined.

**[Table 1]**

| Dilution rate of the antibody (saliva) | Antigen OVM concentration (fmol/spot) | | | | | |
|---|---|---|---|---|---|---|
| | 5 | 10 | 25 | 50 | 75 | 100 |
| × 1 | 703.50 | 1743.80 | 1903.53 | 5334.46 | 5694.00 | 7505.71 |
| × 5 | 448.31 | 732.41 | 1228.44 | 2175.19 | 2346.41 | 2807.67 |
| × 10 | 168.34 | 426.14 | 511.11 | 1246.89 | 1310.56 | 1549.65 |
| × 20 | 111.16 | 253.39 | 351.61 | 731.28 | 597.66 | 820.74 |
| × 30 | 61.64 | 179.26 | 172.30 | 427.99 | 384.04 | 521.34 |
| × 40 | 28.43 | 46.41 | 124.55 | 171.59 | 270.56 | 287.04 |
| × 50 | 6.94 | 83.95 | 58.08 | 259.38 | 186.83 | 321.23 |
| × 100 | 18.29 | 45.49 | 126.91 | 201.51 | 196.97 | 262.43 |

### Example 5

### [Analysis of a sample form a child patient.having food allergy]

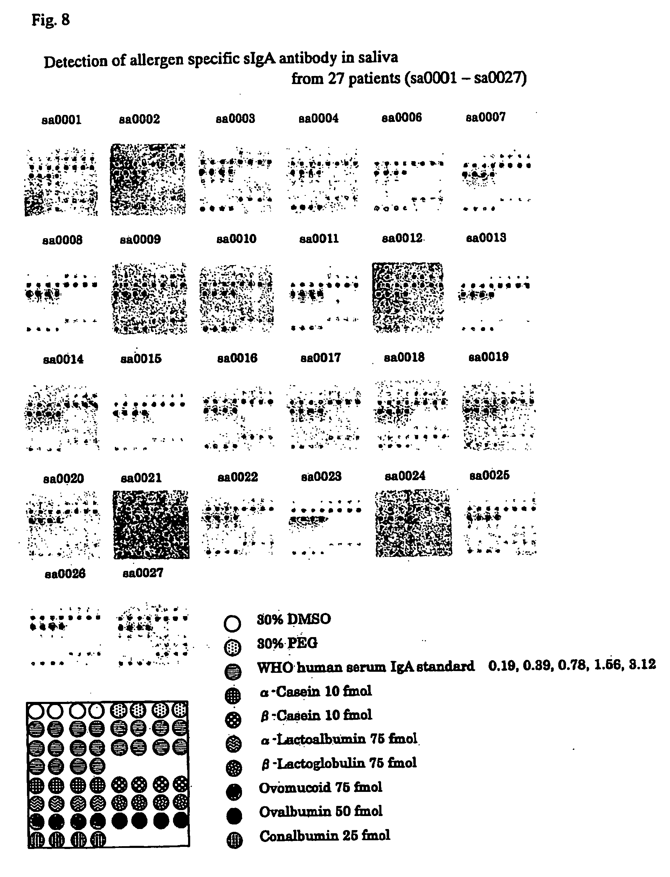

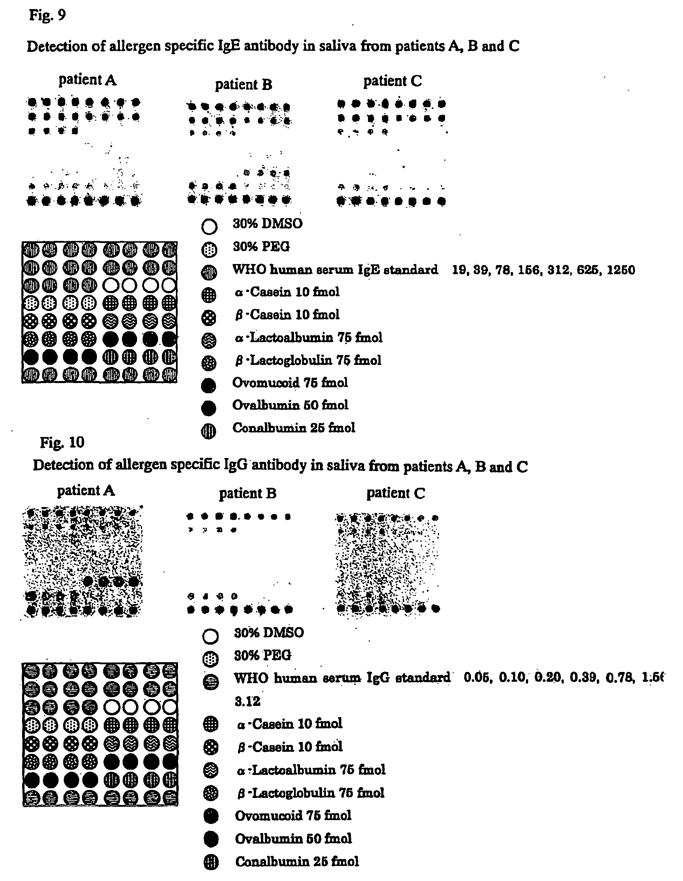

Based on the above basic investigations, samples from child subjects having food allergy were analyzed using a food allergy protein chip. With the acceptance of Ethic Committee of The University of Tokushima, clinical samples from 27 patients who received a sufficient informed consent and gave their acceptance were obtained from Health Insurance Naruto Hospital, to which food (egg and milk) allergy test was carried out. First, in order to test allergen components of egg and milk, 30% DMSO and 30% PEG were spotted as a blank on a Gene Slide, and WHO Human Serum IgE, IgG, IgA were spotted as a standard, and allergen proteins were spotted beneath each of them (n=4). Saliva was diluted 10 fold for reaction, and Cy3-labeled anti-human IgA, IgE, IgG were used as a secondary antibody to detect the fluorescence intensity, and the measurement results are shown (see Figs. 8 to 10). Almost all of the patients reacted to ovoalubumin and conalubumin. However, the reactivity for α-casein, β-casein, α-lactoalubumin, β-lactoglobulin and ovomucoid differed significantly among allergic patients. As such, with the above measurement system using a Gene Slide, it has been shown that a qualitative and quantitative measurement of allergen-specific sIgA, IgE, IgG antibody levels against allergen protein in saliva was possible. Fig. 8 shows the results of qualitative and quantitative measurement of sIgA antibody levels. Fig. 9 shows the results of qualitative and quantitative measurement of allergen-specific IgE antibody levels. Fig. 10 shows the results of qualitative and quantitative measurement of allergen-specific IgG antibody levels. As such, it becomes possible to investigate the allergic conditions of a patient in details with the allergen-specific IgE, IgG, sIgA antibodies against allergen protein in saliva. As a specific example, as it is shown in Figs. 9 and 10, in case of patient A, IgE reacted weakly to α-casein; both IgE and IgG reacted strongly to ovoalubumin, and only IgG reacted strongly to ovomucoid. In case of patient B, reaction of IgE and IgG to ovomucoid and ovoalubumin were observed, and particularly the reaction of IgE was strong. In case of patient C, both IgE and IgG reacted to ovoalubumin, and IgE reacted weakly to conalubumin.

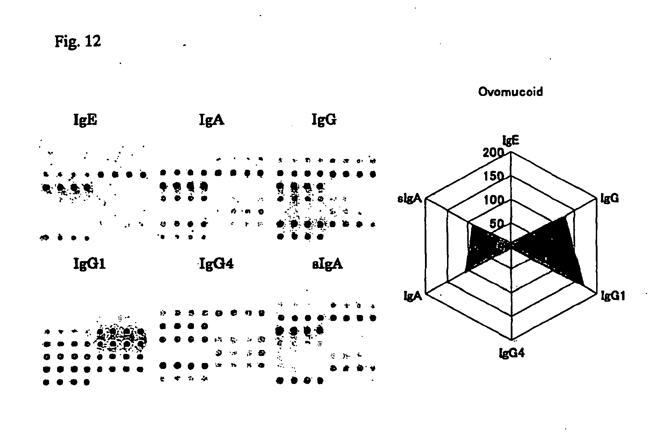

With the assessment of sIgA, IgG, and IgE of allergen-specific saliva of the present invention, and the multilateral assessment of allergen-specific IgG1, IgG4, IgA and IgE from a minute amount of blood (1 to 2 µL) with a Radar chart etc. (Fig. 12) ; there are advantages such as (1) the current allergic conditions can be understood from several perspectives based on evidences; and (2) effectiveness and disease process can be understood from several perspectives.

### Example 6

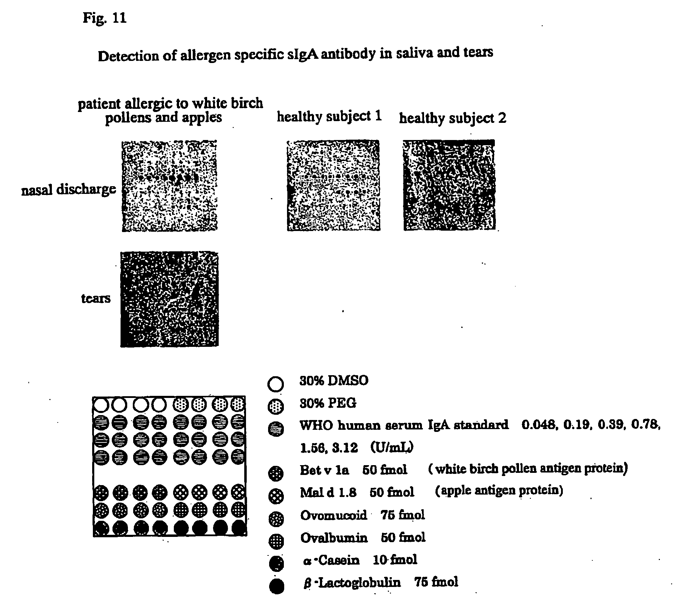

Detection examples using nasal discharge and tears as body fluid other than saliva are shown. Similarly, protein chips spotted with white birch pollen antigen protein (Betv1a), apple antigen protein (Mald1.8) ovomucoid, ovoalubumin, α-casein, and β-lactoglobulin as allergens were prepared. Nasal discharge and tears from patients showing allergic reactions to white birch pollen and apples as well as nasal discharge from 2 normal subjects were collected. Allergen-specific sIgA antibody levels in nasal discharge and tears were measured. As a result, it has been revealed that allergen-specific sIgA antibody levels was possible to be measured with nasal discharge and tears, and that it is useful for symptoms or diagnosis of patients. Specifically, in healthy subject who does not show particular allergic symptom, sIgA antibodies reacting to apple antigen protein are detected, while in patients being allergic to white birch and apple, contrary to healthy subjects, sIgA antibodies to these antigens are not detected or detected in a minute level. That is, contrary to IgE antibody in blood, insufficient formation of sIgA antibody in nasal discharge or tears of allergic patients is thought to induce allergic symptoms. Alternatively, it is suggested that in mucosal membrane where antigens penetrate, sIgA reacting with antigens avoid allergy. Further, in patients showing allergic reactions to white birch pollens and apples, reactions to α-casein are observed in tears, and reactions to β-lactoglobulin are observed in nasal discharge. Trace of allergy in the past or food intolerance was confirmed. As such, it provides useful information for such as analysis of clinical history, food intolerance besides the present conditions of a patient (see Fig. 11).

### Example 7

Further, food allergy protein chips loading with egg and milk allergens were used to measure each antibody level of allergen-specific IgE, IgA, IgG, IgG1, IgG4, and sIgA in a patient's serum and saliva, and the relative levels are shown in a Radar chart (see Fig. 12). It is estimated that the Radar chart shape, or the change in Radar chart shape can be useful to analyze disease conditions, treating history, diagnosis of a patient.

### Industrial Applicability

According to the present invention, even using body fluid other than blood, such as saliva as a sample, a multilateral and global analysis can be conducted even with a minute amount of sample, and particularly, as nonspecific reactions are suppressed as much as possible, it is possible to determine allergic diseases with a high sensitivity and accuracy.

## Claims

1. A method for determining allergic diseases comprising activating a chemically-modified diamond/DLC (Diamond-like Carbon) chip with an activating reagent; conducting a coupling reaction with a peptide comprising allergen or allergen epitope; subsequently contacting a sample with the allergen determination chip to which washing and blocking operations of unreacted active groups have been performed; and detecting an allergen recognizing antibody in the sample captured by the allergen determination chip by an immunoassay using a labelled secondary antibody; wherein a glycine-containing solution is used as a washing solution and a glycine and polyethylene glycol-containing solution is used as a blocking solution used in the washing and blocking operations.

2. The method for determining allergic diseases according to claim 1, wherein a potassium chloride-containing solution is used as a sample diluent.

3. The method for determining allergic diseases according to claim 1 or 2, wherein a potassium chloride-containing solution is used as a diluent of a labelled-secondary antibody.

4. The method for determining allergic diseases according to any one of claims to 3, wherein a Cy3 labelled-secondary antibody is used as a labelled-secondary antibody.

5. The method for determining allergic diseases according to any one of claims 1 to 4, wherein saliva, tears or nasal discharge is used as a sample.

6. The method for determining allergic diseases according to claim 5, wherein saliva, tears or nasal discharge which turbidity at an absorption wavelength of 600 nm is 25 or less is used as a sample.

7. The method for determining allergic diseases according to claim 5, wherein saliva, tears or nasal discharge which has undergone pressure filtration with a low protein-adsorbing filter is used as a sample.

8. The method for determining allergic diseases according to any one of claims 1 to 7, wherein the allergen is a food allergen.

9. An allergy determination chip wherein a chemically modified diamond/DLC (Diamond-like Carbon) chip has been activated with an activating reagent, a coupling reaction has been conducted with a peptide comprising an allergen or allergen epitope, and washing and blocking operations of unreacted activated groups have been conducted using a glycine-containing solution as a washing solution and using a glycine and polyethylene glycol-containing solution as a blocking solution.

10. A kit for determining allergic diseases comprising the allergy determination chip according to claim 9, and a labelled secondary antibody.

11. The kit for determining allergic diseases according to claim 10, wherein the labelled secondary antibody is one or more labelled secondary antibody selected from the group consisting of a labelled anti-slgA antibody, labelled anti-lgG antibody, labelled anti-IgG1 antibody, labelled anti-IgG4 antibody, labelled anti-lgA antibody and labelled anti-lgE antibody.

12. The kit for determining allergic diseases according to claim 10 or 11. further comprising a diluent containing potassium chloride for diluting a sample and/or for diluting a labelled secondary antibody.

13. The kit for determining allergic diseases according to any one of claims 10 to 12, further comprising a low protein-adsorbing filter for pretreating a sample.

## Patentansprüche

1. Verfahren zum Bestimmen von allergischen Erkrankungen, umfassend:
Aktivieren eines chemisch modifizierten Diamant-/DLC-(diamantartigen Kohlenstoff)-Chips mit einem Aktivierungsreagens;
Durchführen einer Kopplungsreaktion mit einem Allergen oder Allergenepitop umfassenden Peptid;
anschließendes Inkontaktbringen einer Probe mit dem Allergen-Bestimmungschip, bezüglich dessen Wasch- und Blockierungsoperationen von nicht reagierten aktiven Gruppen ausgeführt worden sind; und
Detektieren eines Allergen erkennenden Antikörpers in der mittels des Allergen-Bestimmungschips erfassten Probe durch ein Immunoassay, unter Verwendung eines markierten Sekundärantikörpers;
wobei eine Glycin enthaltende Lösung als Waschlösung und eine Glycin und Polyethylenglykol enthaltende Lösung als Blockierlösung bei den Wasch- und Blockieroperationen verwendet werden.

2. Verfahren zum Bestimmen von allergischen Erkrankungen nach Anspruch 1, wobei eine Kaliumchlorid enthaltende Lösung als Probenverdünner verwendet wird.

3. Verfahren zum Bestimmen von allergischen Erkrankungen nach Anspruch 1 oder 2, wobei eine Kaliumchlorid enthaltende Lösung als Verdünner eines markierten Sekundärantikörpers verwendet wird.

4. Verfahren zum Bestimmen von allergischen Erkrankungen nach einem der Ansprüche 1 bis 3, wobei ein Cy3 markierter Sekundärantikörper als markierter Sekundärantikörper verwendet wird.

5. Verfahren zum Bestimmen von allergischen Erkrankungen nach einem der Ansprüche 1 bis 4, wobei Speichel, Tränen oder Nasenausfluss als Probe verwendet wird bzw. werden.

6. Verfahren zum Bestimmen von allergischen Erkrankungen nach Anspruch 5, wobei Speichel, Tränen oder Nasenausfluss, dessen bzw. deren Trübung 25 oder weniger bei einer Absorptionswellenlänge von 600nm beträgt, als Probe verwendet wird bzw. werden.

7. Verfahren zum Bestimmen von allergischen Erkrankungen nach Anspruch 5, wobei Speichel, Tränen oder Nasenausfluss, der bzw. die einer Druckfilterung mit einem Protein niedrigen Gehalts absorbierenden Filter unterzogen ist bzw. sind, als Probe verwendet wird bzw. werden.

8. Verfahren zum Bestimmen von allergischen Erkrankungen nach einem der Ansprüche 1 bis 7, wobei das Allergen ein Nahrungsmittelallergen ist.

9. Allergie-Bestimmungschip, bei dem ein chemisch modifizierter Diamant-DLC-(diamantartiger Kohlenstoff)-Chip mit einem Aktivierungsreagens aktiviert worden ist, eine Kopplungsreaktion mit einem Allergen oder Allergenepitop umfassenden Peptid durchgeführt worden ist, und
Wasch- und Blockieroperationen von nicht reagierten aktivierten Gruppen unter Verwendung einer Glycin enthaltenden Lösung als Waschlösung und unter Verwendung einer Glycin und Polyethylenglykol enthaltenden Lösung als Blockierlösung durchgeführt worden sind.

10. Kit zum Bestimmen von allergischen Erkrankungen, umfassend den Allergie-Bestimmungschip nach Anspruch 9 und einen markierten Sekundärantikörper.

11. Kit zum Bestimmen von allergischen Erkrankungen nach Anspruch 10, wobei der markierte Sekundärantikörper durch einen oder mehrere markierte Sekundärantikörper gebildet ist, die aus der Gruppe ausgewählt sind, bestehend aus einem markierten Anti-sigA-Antikörper, einem markierten Anti-IgG-Antikörper, einem markierten Anti-IgG 1 -Antikörper, einem markierten Anti-IgG4-Antikörper, einem markierten Anti-IgA-Antikörper und einem markierten Anti-IgE-Antikörper.

12. Kit zum Bestimmen von allergischen Erkrankungen nach Anspruch 10 oder 11, ferner umfassend einen Kaliumchlorid enthaltenden Verdünner zum Verdünnen einer Probe und/oder zum Verdünnen eines markierten Sekundärantikörpers.

13. Kit zum Bestimmen von allergischen Erkrankungen nach einem der Ansprüche 10 bis 12, ferner umfassend einen Protein niedrigen Gehalts absorbierenden Filter zum Vorbehandeln einer Probe.

## Revendications

1. Procédé de détermination de maladies allergiques comprenant une activation d'une puce de diamant/DLC (carbone adamantin) chimiquement modifiée avec un activant ; une réalisation d'une réaction de couplage avec un peptide comprenant un allergène ou un épitope d'allergène ; une mise en contact de manière consécutive d'un échantillon avec la puce de détermination d'allergène sur laquelle des opérations de lavage et de blocage de groupes actifs n'ayant pas réagi ont été effectuées ; et une détection d'un anticorps reconnaissant un allergène dans l'échantillon prélevé par la puce de détermination d'allergène par un immunoessai utilisant un anticorps secondaire marqué ; dans lequel une solution contenant de la glycine est utilisée comme solution de lavage et une solution contenant de la glycine et du polyéthylèneglycol est utilisée comme solution de blocage utilisée dans les opérations de lavage et de blocage.

2. Procédé de détermination de maladies allergiques selon la revendication 1, dans lequel une solution contenant du chlorure de potassium est utilisée comme diluant d'échantillon.

3. Procédé de détermination de maladies allergiques selon la revendication 1 ou 2, dans lequel une solution contenant du chlorure de potassium est utilisée comme diluant d'un anticorps secondaire marqué.

4. Procédé de détermination de maladies allergiques selon l'une quelconque des revendications 1 à 3, dans lequel un anticorps secondaire marqué Cy3 est utilisé comme anticorps secondaire marqué.

5. Procédé de détermination de maladies allergiques selon l'une quelconque des revendications 1 à 4, dans lequel de la salive, des larmes ou un écoulement nasal est utilisé comme échantillon.

6. Procédé de détermination de maladies allergiques selon la revendication 5, dans lequel de la salive, des larmes ou un écoulement nasal, dont la turbidité à une longueur d'onde d'absorption de 600 nm est de 25 ou moins, est utilisé comme échantillon.

7. Procédé de détermination de maladies allergiques selon la revendication 5, dans lequel de la salive, des larmes ou un écoulement nasal qui a subi une filtration sous pression avec un filtre adsorbant les petites protéines est utilisé comme échantillon.

8. Procédé de détermination de maladies allergiques selon l'une quelconque des revendications 1 à 7, dans lequel l'allergène est un allergène alimentaire.

9. Puce de détermination d'allergie où une puce de diamant/DLC (carbone adamantin) chimiquement modifiée a été activée avec un activant, une réaction de couplage a été réalisée avec un peptide comprenant un allergène ou un épitope d'allergène, et des opérations de lavage et de blocage de groupes activés n'ayant pas réagi ont été réalisées en utilisant une solution contenant de la glycine comme solution de lavage et en utilisant une solution contenant de la glycine et du polyéthylèneglycol comme solution de blocage.

10. Kit de détermination de maladies allergiques comprenant la puce de détermination d'allergène selon la revendication 9, et un anticorps secondaire marqué.

11. Kit de détermination de maladies allergiques selon la revendication 10, dans lequel l'anticorps secondaire marqué est un ou plusieurs anticorps secondaires marqués sélectionnés dans le groupe constitué d'un anticorps anti-sigA marqué, d'un anticorps anti-IgG marqué, d'un anticorps anti-IgG1 marqué, d'un anticorps anti-IgG4 marqué, d'un anticorps anti-IgA marqué et d'un anticorps anti-IgE marqué.

12. Kit de détermination de maladies allergiques selon la revendication 10 ou 11, comprenant en outre un diluant contenant du chlorure de potassium destiné à diluer un échantillon et/ou à diluer un anticorps secondaire marqué.

13. Kit de détermination de maladies allergiques selon l'une quelconque des revendications 10 à 12, comprenant en outre un filtre adsorbant les petites protéines destiné à prétraiter un échantillon.
